# EUROPÄISCHE PATENTSCHRIFT

(11) **EP 4 518 757 B1**
(45) Veröffentlichungstag und Bekanntmachung des Hinweises auf die Patenterteilung: **25.02.2026**
(21) Anmeldenummer: 24740398.3
(22) Anmeldetag: 04.07.2024
(51) Int. Cl.: A61B 5/22, A61B 5/00

(54) **ANZIEHBARES MESSGERÄT, HILFSSYSTEM UMFASSEND EIN ANZIEHBARES MESSGERÄT, VERFAHREN ZUM ERMITTELN EINER MUSKELHÄRTE EINES SKELETTMUSKELS**
PULL-ON MEASURING INSTRUMENT, AUXILIARY SYSTEM COMPRISING A PULL-ON MEASURING INSTRUMENT, METHOD FOR DETERMINING MUSCULAR HARDENING OF A SKELETAL MUSCLE
INSTRUMENT DE MESURE À TRACTION, SYSTÈME AUXILIAIRE COMPRENANT UN INSTRUMENT DE MESURE DE TRACTION, PROCÉDÉ DE DÉTERMINATION DE LA TENSION MUSCULAIRE D'UN MUSCLE SQUELETTIQUE

(30) Priorität: 27.07.2023 DE 102023119950
(43) Veröffentlichungstag der Anmeldung: 12.03.2025
(73) Patentinhaber: J. Schmalz GmbH, 72293 Glatten (DE)
(72) Erfinder: SINGER, Raphael, 72160 Horb am Neckar (DE)
(74) Vertreter: DREISS Patentanwälte PartG mbB
(86) Internationale Anmeldenummer: PCT/EP2024/068945
(87) Internationale Veröffentlichungsnummer: WO 2025/021472

(56) Entgegenhaltungen:
- JP-U- 3 028 586
- US-A- 5 879 312

## Beschreibung

Die vorliegende Erfindung betrifft ein anziehbares Messgerät, das eine Messeinrichtung mit wenigstens zwei Messeinheiten und eine Halteeinrichtung zum Anbringen der Messeinrichtung an einem Körperteil einer Person umfasst. Außerdem betrifft die vorliegende Erfindung ein Hilfssystem, das ein solches Messgerät und ein anziehbares Exoskelett umfasst. Ferner betrifft die vorliegende Erfindung ein Verfahren zum Ermitteln einer Muskelhärte eines Skelettmuskels einer Person.

Anziehbare Exoskelette können dazu verwendet werden, Belastungen auf einen Benutzer, bspw. beim Anheben von schweren Gegenständen, entweder direkt in den Boden zu übertragen oder zumindest durch ausgewählte Körperbereiche des Benutzers zu leiten. Auf diese Weise kann das Gelenk- und Muskelsystem des Benutzers geschont werden.

Um eine wirksame Entlastung eines Benutzers durch ein Exoskelett zu realisieren, ist es notwendig, einen Unterstützungsbedarf des Benutzers zu ermitteln. Beispielsweise ist es bekannt, einen Skelettmuskel des Benutzers zu überwachen und daraus den Unterstützungsbedarf abzuleiten. Herkömmlicherweise werden zum Überwachen eines Skelettmuskels anziehbare Messgeräte eingesetzt. Solche Messgeräte umfassen eine Messeinrichtung zum Überwachen des Skelettmuskels und eine Halteeinrichtung zum Anbringen der Messeinrichtung an einem den Skelettmuskel aufweisenden Körperteil. Diesbezüglich offenbaren bspw. die Druckschriften WO 2018 050 191 A1 und ES 2 335 337 B1 anziehbare Messgeräte in Form von Kraftsensorarmbändern. Die Druckschriften CN 111 150 373 B und KR 101 675 577 B1 offenbaren anziehbare Messgeräte in Form von Gasdrucksensorarmbändern.

Bekannte Kraftsensorarmbänder und Gasdrucksensorarmbänder haben jedoch den Nachteil, dass das Messergebnis durch verschiedene Effekte verfälscht wird. Beispielsweise hat der Grad der Festigkeit des Anlegens der Armbänder einen Einfluss auf das Messergebnis. Zudem wird das Messergebnis auch durch Ausbauchungen des überwachten Skelettmuskels beeinträchtigt, die beim Bewegen entstehen und mit einer Umfangsänderung einhergehen.

Die Erfindung beschäftigt sich mit der Aufgabe, bei einem anziehbaren Messgerät die Überwachung des Skelettmuskels zu verbessern.

Diese Aufgabe wird erfindungsgemäß durch ein anziehbares Messgerät mit den Merkmalen des Anspruchs 1 gelöst.

Die Unteransprüche und die Beschreibung geben vorteilhafte Varianten und Ausführungsformen an.

Erfindungsgemäß ist also ein anziehbares Messgerät vorgesehen. Das Messgerät umfasst eine Messeinrichtung, die wenigstens zwei Messeinheiten zum Überwachen eines Skelettmuskels einer Person umfasst. Das Messgerät umfasst außerdem eine Halteeinrichtung zum Anbringen der Messeinrichtung an einem den Skelettmuskel aufweisenden Körperteil der Person. Vorzugsweise ist der Körperteil eine Extremität der Person, insbesondere ein Arm oder ein Bein.

Es ist nun vorgesehen, dass die Messeinheiten jeweils einen Kraftsensor und eine zugeordnete Federeinheit umfassen, wobei die Federeinheiten jeweils voneinander verschiedene Federkonstanten aufweise. Die Messeinheiten sind derart an dem Körperteil anbringbar, dass die Federeinheiten durch einen jeweils anderen Abschnitt des Skelettmuskels mit Kraft beaufschlagbar, bzw. spannbar sind, und dass die Kraftsensoren dazu ausgebildet sind, eine Spannkraft der zugeordneten Federeinheit zu erfassen.

Der Erfindung liegt der Ansatz zugrunde, dass ein Skelettmuskel als ein Raster bzw. ein Array von Federeinheiten mit gleicher Federkonstante angesehen werden kann. Die Stärke bzw. der Wert der Federkonstante des Skelettmuskels variiert mit dem Grad der Anspannung des Skelettmuskels. Werden die erfindungsgemäßen Messeinheiten derart an dem Körperteil angebracht, dass die Federeinheiten durch einen jeweils anderen Abschnitt des Skelettmuskels spannbar sind, so bilden die Federeinheiten mit dem betreffenden, zugeordneten Abschnitt des Skelettmuskels jeweils eine Federanordnung mit einer Ersatzfederkonstante. Weil sich die Federkonstanten der Federeinheiten voneinander unterscheiden, unterscheiden sich auch die Ersatzfederkonstanten der gebildeten Federanordnungen voneinander. Wird der überwachte Skelettmuskel angespannt, so wird auf die Federeinheit mit der größeren Federkonstante eine größere Spannkraft übertragen als auf die Federeinheit mit der geringeren Federkonstante. Anhand der durch die Kraftsensoren erfassten Spannkräfte kann dann die Muskelhärte des Skelettmuskels als Federkonstante ermittelt bzw. geschätzt werden. Im idealisierten Fall, wonach der Skelettmuskel eine lineare Federkennlinie aufweist, wäre eine Berechnung der Federkonstante des Skelettmuskels möglich. Im realen Fall wird aufgrund verschiedener Effekte eher eine Schätzung, jedoch mit ausreichender Genauigkeit, realisiert. Durch diesen Ansatz können die zuvor im Zusammenhang mit Kraftsensorarmbändern und Gasdrucksensorarmbändern genannten Nachteile überwunden werden. Folglich kann aus der Federkonstante des Skelettmuskels der Unterstützungsbedarf des Benutzers nutzerfreundlich und zuverlässig hergeleitet werden. Dabei ist die Ermittlung der Federkonstante des Skelettmuskels weitestgehend unabhängig von Einflussgrößen wie beispielsweise der Person, die das Messgerät verwendet, oder dem Zeitpunkt bzw. der Muskelanspannung, zu dem das Messgerät angelegt wird. Bei einer gleichhohen Muskelanspannung kann unabhängig von derartigen Einflussgrößen stets eine vergleichbare Federkonstante ermittelt werden.

Vorzugsweise beträgt die Federkonstante der einen Federeinheit zumindest das 1,1-fache der Federkonstante der anderen Federeinheit. Durch eine solche Ausbildung der Federeinheiten wird erreicht, dass sich die Sensorsignale der Kraftsensoren für eine besonders präzise Ermittlung der Federkonstante des Skelettmuskels ausreichend stark voneinander unterscheiden.

Vorzugsweise umfassen die erfindungsgemäßen Federeinheiten jeweils nur ein einziges Federelement. Die Federeinheiten können jedoch auch jeweils mehrere Federelemente umfassen, die beispielsweise in Reihe oder parallel zueinander geschaltet sind.

Die erfindungsgemäßen Kraftsensoren sind dazu ausgebildet, eine Spannkraft der zugeordneten Federeinheit zu erfassen, also eine Spannkraft der Federeinheit derselben Messeinheit. Je nach Ausbildung der Messeinheiten können die Kraftsensoren die Spannkraft als Zugkraft oder als Druckkraft erfassen. Im Rahmen der Offenbarung sind mit dem Begriff "Kraft" sowohl eine Kraft im engeren Sinne als auch eine Flächenpressung und ein Gasdruck gemeint. Folglich können die erfindungsgemäßen Kraftsensoren die Spannkraft zumindest in einigen Ausführungsformen auch als Flächenpressung oder als Gasdruck erfassen.

Insbesondere umfasst das Messgerät nur eine einzige Messeinrichtung, sodass mittels des Messgeräts dann auch nur ein einziger Skelettmuskel der Person bestimmungsgemäß überwacht werden kann. Das Messgerät kann jedoch auch mehrere Messeinrichtungen umfassen, die dann einem jeweils anderen Skelettmuskel desselben Körperteils der Person zuordenbar sind. Dadurch wird es ermöglicht, mittels desselben Messgeräts gleichzeitig mehrere verschiedene Skelettmuskel desselben Körperteils zu überwachen. Ist der Körperteil rein beispielhalber ein Arm, so können durch ein Messgerät mit zwei Messeinrichtungen gleichzeitig beispielsweise sowohl der Musculus biceps brachii als auch der Musculus triceps brachii überwacht werden.

Erfindungsgemäß umfasst das Messgerät eine Auswerteeinheit, die dazu ausgebildet ist, in Abhängigkeit von Sensorsignalen der Kraftsensoren eine Federkonstante des Skelettmuskels zu ermitteln. Beispielsweise ermittelt die Auswerteeinheit die Federkonstante gemäß einer Lookup-Tabelle, gemäß einem Kennfeld, gemäß einer Rechenvorschrift oder gemäß einem Rechennetzwerk. In einigen alternativen Ausführungsformen ist das Messgerät selbst frei von einer Auswerteeinheit. Es kann dann eine bezogen auf das Messgerät externe Auswerteeinheit vorhanden sein, die mit den Kraftsensoren kommunikationstechnisch verbunden und dazu ausgebildet ist, in Abhängigkeit von den Sensorsignalen der Kraftsensoren die Federkonstante des Skelettmuskels zu ermitteln.

Im Hinblick auf die Ausbildung der Federeinheiten sind verschiedene Ausführungen möglich. Vorzugsweise umfasst wenigstens eine der Federeinheiten eine Metallfeder oder eine Elastomerfeder. Durch die Verwendung solcher Federn kann die Messeinrichtung kostengünstig realisiert werden.

Im Hinblick auf das Material der Federeinheiten sind solche Materialien bevorzugt, die eine lineare oder zumindest im Wesentlichen lineare Federkennlinie ermöglicht. Die Federeinheiten können beispielsweise aus einem textilen Material oder aus einem Metallwerkstoff gefertigt sein. Die Federkonstante der Federeinheiten ist bevorzugt in der Größenordnung der Federkonstante des zu überwachenden Skelettmuskels.

Auch im Hinblick auf die Form der Federeinheiten sind verschiedene Ausführungen möglich. Vorzugsweise ist wenigstens eine der Federeinheiten zylinderförmig, spiralförmig, tellerförmig, balkenförmig, bandförmig oder strangförmig ausgebildet. Derartige Federeinheiten sind kostengünstig erhältlich. Aufgrund der kompakten Ausbildung kann zudem auch ein insgesamt kompaktes Messgerät realisiert werden. Dies geht mit einem hohen Tragekomfort für den Benutzer einher.

In einigen bevorzugten Ausführungsformen umfasst wenigstens eine der Federeinheiten eine Gasdruckfeder. Gasdruckfedern haben den Vorteil, dass sie eine Federkennlinie mit einem annähernd linearen Verlauf aufweisen. Eine Federkennlinie mit einem linearen bzw. annähern linearen Verlauf ermöglicht eine besonders präzise Ermittlung der Federkonstante des Skelettmuskels.

Auch im Hinblick auf die Art der Kraftsensoren sind verschiedene Ausführungen möglich. Vorzugsweise umfasst wenigstens einer der Kraftsensoren wenigstens einen kraftabhängigen elektrischen Widerstand, wenigstens eine kraftabhängige elektrische Kapazität, wenigstens eine kraftabhängige elektrische Induktivität oder wenigstens ein Piezoelement. Kraftsensoren mit kraftabhängigem elektrischen Widerstand sind auch als FSR-Sensoren (Force Sensing Resistors) oder als DMS Elemente (Dehnmessstreifen) bekannt. Kraftabhängige elektrische Widerstände, Kapazitäten, Induktivitäten oder Piezoelemente zeichnen sich durch ihre geringe Bauhöhe aus, sodass durch die Verwendung von kraftabhängigen elektrischen Widerständen, Kapazitäten, Induktivitäten oder Piezoelementen ein besonders kompaktes Messgerät realisiert werden kann.

In einigen bevorzugten Ausführungsformen ist vorgesehen, dass die Federeinheiten einen ersten Endabschnitt und einen zweiten Endabschnitt aufweisen, wobei der zugeordnete Kraftsensor durch den ersten Endabschnitt mit der Federeinheit wirkverbunden ist, und wobei die Federeinheit durch den zweiten Endabschnitt mit dem Skelettmuskel wirkverbindbar oder wirkverbunden ist. Bei bestimmungsgemäßer Anordnung des Messgeräts sind also die Federeinheiten zwischen dem Skelettmuskel und den Kraftsensoren angeordnet. Diese Anordnung der Kraftsensoren hat den Vorteil, dass eine kommunikationstechnische Anbindung der Kraftsensoren erleichtert wird, insbesondere eine kommunikationstechnische Anbindung an die zuvor erwähnte Auswerteeinheit.

In einigen bevorzugten Ausführungsformen ist vorgesehen, dass die Federeinheiten einen ersten Endabschnitt und einen zweiten Endabschnitt aufweisen, wobei der zugeordnete Kraftsensor durch den ersten Endabschnitt mit der Federeinheit wirkverbunden ist, und wobei die Federeinheit durch den Kraftsensor mit dem Skelettmuskel wirkverbindbar oder wirkverbunden ist. Bei bestimmungsgemäßer Anordnung des Messgeräts sind also die Kraftsensoren zwischen dem Skelettmuskel und den Federeinheiten angeordnet. Auch bei einer solchen Anordnung der Federeinheiten sind die Federeinheiten durch den Skelettmuskel spannbar.

In einigen bevorzugten Ausführungsformen ist vorgesehen, dass die Messeinrichtung wenigstens drei Messeinheiten umfasst. Durch die Erhöhung der Anzahl an Messeinheiten können die Robustheit und/oder die Präzision im Hinblick auf das Ermitteln der Federkonstante des Skelettmuskels gesteigert werden. Insbesondere weisen die Federeinheiten der Messeinheiten eine jeweils andere Federkonstante auf. Alternativ dazu weisen die Federeinheiten von wenigstens zwei Messeinheiten die gleiche Federkonstante auf. Wenigstens eine der Messeinheiten ist demnach redundant und kann beispielsweise zur Plausibilisierung der Sensorsignale der übrigen Messeinheiten genutzt werden.

In einigen bevorzugten Ausführungsformen ist vorgesehen, dass die Halteeinrichtung ein Halteband, eine Haltemanschette oder eine Haltebandage umfasst. Solche Haltereinrichtungen ermöglichen eine sichere und präzise Anbringung der Messeinrichtung an dem Körperteil. Zudem gehen solche Halteeinrichtungen mit einem hohen Tragekomfort für den Benutzer einher.

In einigen bevorzugten Ausführungsformen ist vorgesehen, dass die Halteeinrichtung eine insbesondere starre Tragstruktur umfasst, wobei die Messeinheiten an der Tragstruktur angeordnet sind. Durch die Anordnung der Messeinheiten an der gemeinsamen Tragstruktur wird die bestimmungsgemäße Anbringung der Messeinheiten an dem Körperteil erleichtert. Besonders bevorzugt ist die Tragstruktur an dem Halteband, der Haltemanschette bzw. der Haltebandage insbesondere lösbar befestigt.

In einigen bevorzugten Ausführungsformen ist vorgesehen, dass sich die Federeinheiten bei bestimmungsgemäßer Anordnung des Messgeräts radial zu dem Körperteil erstrecken. Bei einer solchen Ausrichtung der Federeinheiten werden die Federeinheiten durch den Skelettmuskel mit einer in Längserstreckung der Federeinheiten wirkenden Spannkraft beaufschlagt. Insbesondere sind die Messeinheiten bei dieser Ausführungsform nebeneinander an der zuvor erwähnten Tragstruktur angeordnet, wobei die Federeinheiten sich in dieselbe Richtung von der Tragstruktur wegerstrecken.

In einigen bevorzugten Ausführungsform ist vorgesehen, dass sich die Federeinheiten bei bestimmungsgemäßer Anordnung des Messgeräts in Umfangsrichtung des Körperteils entlang des Skelettmuskels erstrecken. Die beiden Enden derselben Federeinheit sind bevorzugt in Umfangsrichtung hintereinander angeordnet. Auch eine solche Ausbildung des Messgeräts ermöglicht eine ausreichend präzise Ermittlung bzw. Schätzung der Federkonstante des Skelettmuskels. Vorzugsweise sind die Federeinheiten derart angeordnet, dass sie bei bestimmungsgemäßer Anordnung des Messgeräts bezogen auf die Längsmittelachse des Körperteils zueinander axial versetzt sind. Um die Spannkraft der Federeinheiten zu erfassen, können die Kraftsensoren jeweils mit einem der Enden der zugeordneten Federeinheit wirkverbunden sein. Besonders bevorzugt sind die Federeinheiten bei dieser Ausführungsform als insbesondere textile elastische Bänder ausgebildet.

Die zu lösende Aufgabe wird auch durch ein Hilfssystem mit den Merkmalen des Anspruchs 13 gelöst. Das Hilfssystem umfasst ein anziehbares Exoskelett mit wenigstens einem ansteuerbaren Aktuator. Außerdem umfasst das Hilfssystem ein wie vorstehend beschrieben ausgebildetes Messgerät. Das Hilfssystem umfasst ferner eine Steuereinrichtung, die dazu ausgebildet ist, den Aktuator des Exoskeletts in Abhängigkeit von durch das Messgerät bereitstellbaren Informationen anzusteuern. Die besagten Informationen umfassen insbesondere eine durch das Messgerät ermittelte bzw. geschätzte Federkonstante eines Skelettmuskels, dem das Messgerät zugeordnet ist.

Hinsichtlich der mit dem Hilfssystem erzielbaren Vorteile wird auf die diesbezüglichen Ausführungen zum Messgerät verwiesen. Zur weiteren Ausgestaltung des Hilfssystems können die im Zusammenhang mit dem Messgerät beschriebenen Merkmale dienen.

Es sei darauf hingewiesen, dass das Messgerät nicht auf den Einsatz in einem Hilfssystem mit einem Exoskelett beschränkt ist. Das Messgerät kann beispielsweise auch als Teil eines digitalen Ergonomiebewertungsgeräts oder im Trainingsbereich zur Nachverfolgung einer Veränderung der Muskelfitness eingesetzt werden.

Die zu lösende Aufgabe wird auch durch ein Verfahren mit den Merkmalen des Anspruchs 14 gelöst, das zum Ermitteln einer Muskelhärte eines Skelettmuskels einer Person dient. Dabei ist vorgesehen, dass wenigstens zwei Federeinheiten, die sich in ihrer Federkonstante voneinander unterscheiden, derart an einem den Skelettmuskel aufweisenden Körperteil der Person angebracht werden, dass die Federeinheiten durch einen jeweils anderen Abschnitt des Skelettmuskels spannbar sind, dass die Spannkräfte der Federeinheiten insbesondere laufend erfasst werden, und dass in Abhängigkeit von den erfassten Spannkräften als Muskelhärte eine Federkonstante des Skelettmuskels ermittelt wird.

Vorzugsweise wird das Verfahren durch ein wie vorstehend beschrieben ausgebildetes anziehbares Messgerät durchgeführt.

Hinsichtlich der mit dem Verfahren erzielbaren Vorteile wird auf die diesbezüglichen Ausführungen zum Messgerät verwiesen. Zur weiteren Ausgestaltung des Verfahrens können die im Zusammenhang mit dem Messgerät beschriebenen Merkmale dienen.

Die Erfindung wird im Folgenden anhand der Figuren näher beschrieben, wobei gleiche oder funktional gleiche Elemente gegebenenfalls lediglich einmal mit Bezugszeichen versehen sind. Die Beschreibung dient als Beispiel und ist nicht einschränkend zu verstehen. Es zeigen
- Figur 1: eine Schnittdarstellung eines Körperteils mit einem daran angebrachten Messgerät,
- Figur 2: eine weitere Schnittdarstellung eines Körperteils mit einem daran angebrachten Messgerät,
- Figur 3: eine Schnittdarstellung eines Körperteils mit einem daran angebrachten Messgerät gemäß einem weiteren Ausführungsbeispiel, und
- Figur 4: ein Hilfssystem umfassend ein anziehbares Exoskelett und ein anziehbares Messgerät.

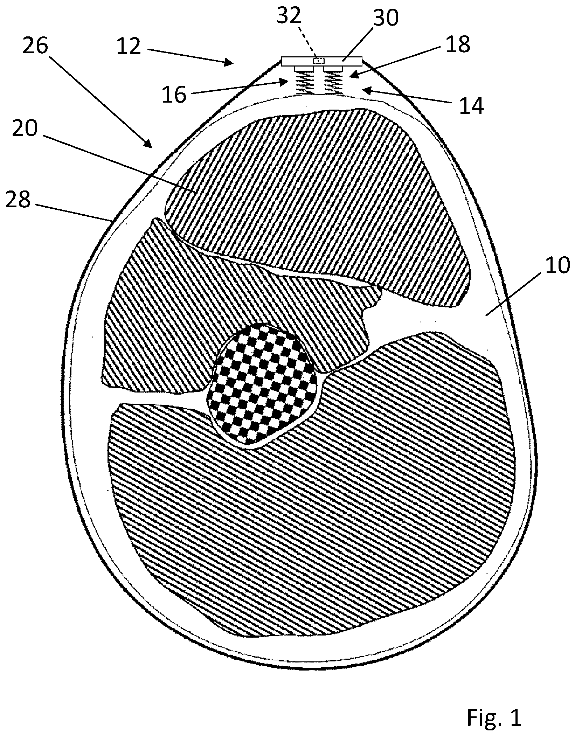

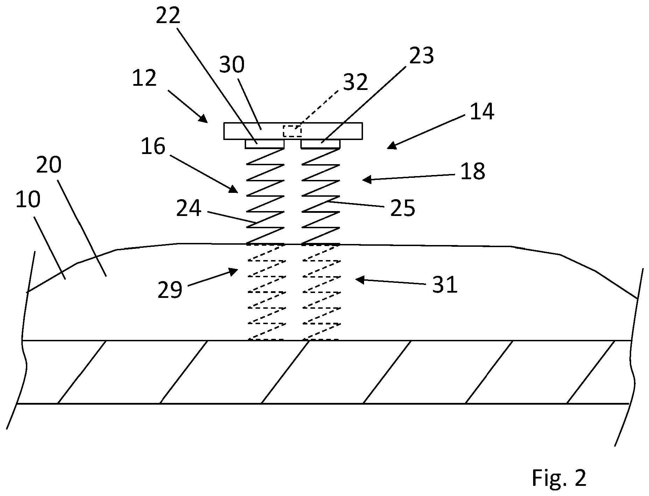

In den Figuren 1 und 2 ist ein Körperteil 10 einer Person mit einem an dem Körperteil 10 angebrachten anziehbaren Messgerät 12 dargestellt. Vorliegend ist der Körperteil 10 ein Arm 10 der Person.

Dabei zeigt Figur 1 einen Querschnitt des Körperteils 10, bei dem die Schnittebene senkrecht zu der Längserstreckung des Körperteils 10 ausgerichtet ist. Figur 2 zeigt einen Längsschnitt des Körperteils 10, bei dem die Schnittebene parallel zu dem Körperteil 10 ausgerichtet ist. Es sei darauf hingewiesen, dass die Figuren 1 und 2 lediglich schematische Darstellungen sind, sodass den Figuren 1 und 2 insbesondere keine Größenverhältnisse zu entnehmen sind.

Das Messgerät 12 umfasst eine Messeinrichtung 14, die zwei Messeinheiten 16 und 18 zum Überwachen eines Skelettmuskels 20 der Person umfasst. In Figur 2 ist die Messeinrichtung 14 gegenüber der Figur 1 um 90° verdreht dargestellt, damit auch in Figur 2 beide Messeinheiten 16 und 18 zu sehen sind. Im Folgenden wird die Messeinheit 16 als erste Messeinheit 16 und die Messeinheit 18 als zweite Messeinheit 18 bezeichnet.

Bei dem dargestellten Ausführungsbeispiel ist der Skelettmuskel 20 der Musculus biceps brachii 20 des Arms 10. Das Messgerät 12 kann jedoch auch zum Überwachen eines anderen Skelettmuskels eingesetzt werden. Zudem kann das Messgerät 12 auch eine weitere Messeinrichtung aufweisen, um gleichzeitig mehrere Skelettmuskeln desselben Körperteils zu überwachen, bspw. den Musculus biceps brachii und den Musculus triceps brachii.

Die erste Messeinheit 16 umfasst einen Kraftsensor 22 und eine dem Kraftsensor 22 zugeordnete Federeinheit 24. Auch die zweite Messeinheit 18 umfasst einen Kraftsensor 23 und eine dem Kraftsensor 23 zugeordnete Federeinheit 25. Die Längen der Federeinheiten 24 und 25 sind bekannt. Im Hinblick auf ihre Federkonstanten unterscheiden sich die Federeinheiten 24 und 25 voneinander. Vorliegend ist die Federkonstante der Federeinheit 24 größer als die Federkonstante der Federeinheit 25. Vorzugsweise unterscheiden sich die Federkonstanten der Federeinheiten 24 und 25 um einen Faktor von wenigstens 1,1 voneinander. Eine der Federkonstanten ist dann also wenigstens 10% größer als die andere der Federkonstanten.

Bei dem dargestellten Ausführungsbeispiel umfassen die Federeinheiten 24 und 25 jeweils eine Schraubenfeder. Eine solche Ausbildung der Federeinheiten 24 und 25 ist bevorzugt. In anderen Ausführungsbeispielen können die Federeinheiten 24 und 25 jedoch auch Tellerfedern, Gasdruckfedern, Balkenfedern oder Elastomerfedern umfassen. Zudem können die Federeinheiten 24 und 25 auch mehr als nur ein einziges Federelement umfassen. Insbesondere umfassen die Federeinheiten 24 und 25 jeweils mehrere Federelemente, die beispielsweise in Reihe oder parallel zueinander geschaltet sind. Mit der Federkonstante der Federeinheiten 24 und 25 ist bei einer solchen Ausbildung die Ersatzfederkonstante gemeint und nicht die Federkonstante eines einzelnen Federelementes.

Die Kraftsensoren 22 und 23 sind dazu ausgebildet, eine Spannkraft der zugeordneten Federeinheit 24 bzw. 25 zu erfassen. Vorliegend umfassen die Kraftsensoren 22 und 23 hierzu jeweils einen kraftabhängigen elektrischen Widerstand.

Das Messgerät 12 umfasst außerdem eine Halteeinrichtung 26 zum Anbringen der Messeinrichtung 14 an dem Körperteil 10. Bei dem vorliegenden Ausführungsbeispiel umfasst die Halteeinrichtung 26 ein verformbares Halteband 28. In Figur 2 liegt das Halteband 28 außerhalb der Schnittebene und ist deshalb nicht erkenntlich.

Zusätzlich zu dem Halteband 28 umfasst die Halteeinrichtung 26 eine starre Tragstruktur 30, die vorliegend plattenförmig ausgebildet ist. Die Messeinheiten 16 und 18 sind nebeneinander an der Tragstruktur 30 angeordnet und erstrecken sich in dieselbe Richtung von der Tragstruktur 30 weg. Vorliegend sind die Kraftsensoren 22 und 23 mit der Tragstruktur 30 direkt verbunden.

Die Messeinrichtung 14 ist mittels der Halteeinrichtung 26 derart an dem Körperteil 10 angebracht, dass die Federeinheiten 24 und 25 durch einen jeweils anderen Abschnitt des Skelettmuskels 20 spannbar sind. Wird der Skelettmuskel 20 angespannt, so beaufschlagen die Abschnitte des Skelettmuskels 20 die Federeinheiten 24 und 25 folglich mit Spannkräften.

Ein Skelettmuskel kann als ein Raster bzw. ein Array von Federeinheiten mit gleicher Federkonstante angesehen werden. Dies ist in Figur 2 durch gestrichelte Federn im Inneren des Skelettmuskels 20 angedeutet. Die Höhe der Federkonstante des Skelettmuskels 20 variiert mit dem Grad der Anspannung des Skelettmuskels. Die Federeinheiten 24 und 25 bilden bei bestimmungsgemäßer Anordnung mit dem Abschnitt des Skelettmuskels 20, durch den sie gespannt werden können, jeweils eine Federanordnung 29 bzw. 31 mit einer Ersatzfederkonstante. Wie zuvor erwähnt wurde, unterscheiden sich die Federeinheiten 24 und 25 im Hinblick auf ihre Federkonstante voneinander. Dies führt dazu, dass sich auch die Federanordnungen 29 und 31 im Hinblick auf ihre Ersatzfederkonstante voneinander unterscheiden. Weil sich die Federkonstanten der Federeinheiten 24 und 25 voneinander unterscheiden, werden die Federeinheiten 24 und 25 beim Anspannen des Skelettmuskels 20 mit einer jeweils anderen Spannkraft beaufschlagt. Konkret wird die Federeinheit 24 mit der größeren Federkonstante mit einer größeren Spannkraft beaufschlagt als die Federeinheit 25 mit der geringeren Federkonstante. Entsprechend unterscheiden sich auch die durch die Kraftsensoren 22 und 23 erfassten Spannkräfte in ihrer Höhe voneinander.

Das Messgerät 12 umfasst außerdem eine Auswerteeinheit 32, die vorliegend in die Tragstruktur 30 integriert ist. Die Auswerteeinheit 32 ist dazu ausgebildet, in Abhängigkeit von Sensorsignalen der Kraftsensoren 22 und 23 eine Federkonstante des Skelettmuskels 20 zu ermitteln. Die Federkonstante des Skelettmuskels 20 beschreibt eine Muskelhärte des Skelettmuskels 20. Beispielsweise ermittelt die Auswerteeinheit 32 die Federkonstante des Skelettmuskels 20 gemäß einer Lookup-Tabelle, gemäß einem Kennfeld, gemäß einer Rechenvorschrift oder gemäß einem Rechennetzwerk.

Die Federeinheiten 24 und 25 weisen jeweils einen ersten Endabschnitt und einen zweiten Endabschnitt auf. Die Endabschnitte sind Axialabschnitte der Federeinheiten 24 und 25. Bei dem in den Figuren dargestellten Ausführungsbeispiel sind die Federeinheiten 24 und 25 jeweils durch die ersten Endabschnitte mit den Kraftsensoren 22 bzw. 23 wirkverbunden. Durch die freien zweiten Endabschnitte sind die Federeinheiten 24 und 25 mit dem Skelettmuskel 20 wirkverbunden.

Wie aus Figur 1 erkenntlich ist, erstrecken sich die Federeinheiten 24 und 25 vorliegend radial zu dem Körperteil 10. In Anbetracht dieser Ausrichtung der Federeinheiten 24 und 25 beaufschlagt der Skelettmuskel 20 die Federeinheiten 24 und 25 mit Spannkräften, die in Längserstreckung der Federeinheiten 24 und 25 auf die Federeinheiten 24 und 25 wirken. Die Federeinheiten 24 und 25 werden durch die Spannkräfte gestaucht.

Bei dem in Figur 1 dargestellten Ausführungsbeispiel stehen die Federeinheiten 24 und 25 in direkter Berührung mit der Haut des Benutzers. Abweichend davon kann das Messgerät 12 auch über der Kleidung getragen werden, wodurch der Tragekomfort für den Benutzer gesteigert wird.

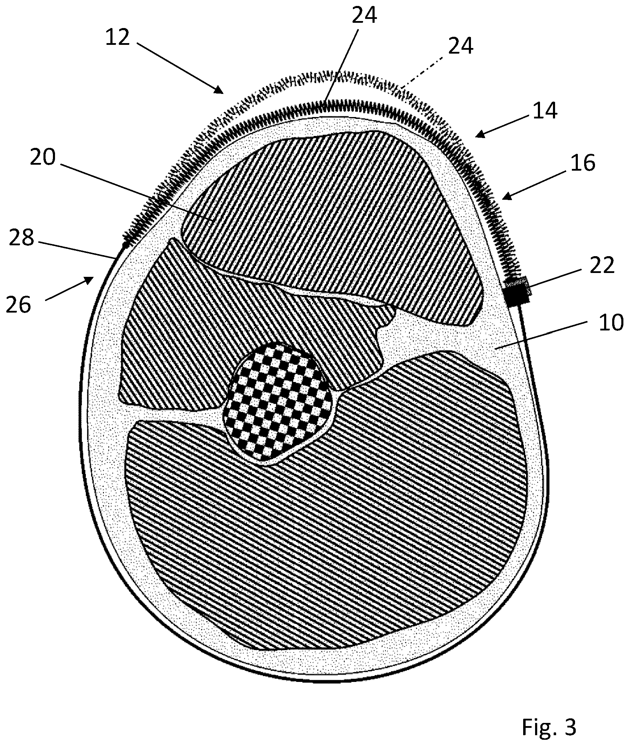

Figur 3 zeigt ein anziehbares Messgerät 12 gemäß einem weiteren Ausführungsbeispiel. Bei dem in Figur 3 dargestellten Messgerät 12 erstrecken sich die Federeinheiten 24, 25 in Umfangsrichtung des Körperteils 10 entlang des Skelettmuskels 20. Es sei darauf hingewiesen, dass in Figur 3 nur die Messeinheit 16 zu sehen ist. Die andere Messeinheit 18 ist bezogen auf die Längsmittelachse des Körperteils 10 axial versetzt zu der Messeinheit 16 angeordnet und befindet sich deshalb außerhalb der Bildebene. Die Federeinheit 24 ist in Figur 3 vereinfacht als Schraubenfeder dargestellt. Es kann sich bei der Federeinheit 24 jedoch auch beispielsweise um ein textiles, elastisches Band handeln. Wird der überwachte Skelettmuskel 20 gespannt, so werden die Federeinheiten 24, 25 mit einer radial nach außen wirkenden Spannkraft beaufschlagt und dadurch gedehnt. Dieser gedehnte Zustand der Federeinheit 24 ist in Figur 3 gestrichelt angedeutet.

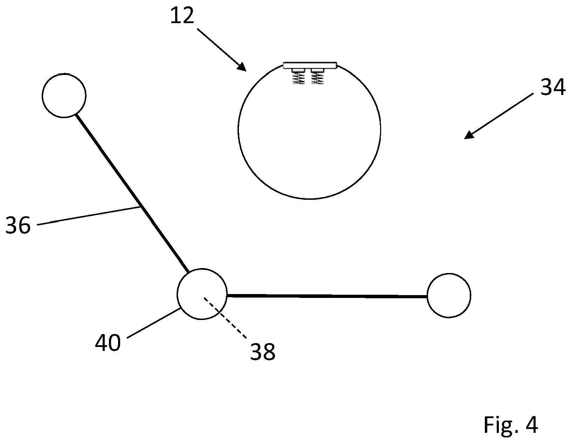

Figur 4 zeigt eine schematische Darstellung eines Hilfssystems 34. Das Hilfesystem 34 umfasst vorliegend beispielhalber das zuvor beschriebene und in Figur 1 dargestellte anziehbare Messgerät 12. Außerdem umfasst das Hilfssystem 34 ein anziehbares Exoskelett 36, das in Figur 3 lediglich vereinfacht dargestellt ist. Das Exoskelett 36 umfasst einen ansteuerbaren Aktuator 38, der vorliegend in ein Gelenk 40 des Exoskeletts 36 integriert ist.

Ferner ist eine in den Figuren nicht dargestellte Steuereinrichtung vorhanden. Die Steuereinrichtung ist dazu ausgebildet, den Aktuator 38 in Abhängigkeit von durch das Messgerät 12 bereitstellbaren Informationen anzusteuern. Insbesondere ist auch die Steuereinrichtung in das Gelenk 40 integriert. Die Steuereinrichtung kann jedoch auch bezogen auf das Exoskelett 36 extern ausgeführt sein. Insbesondere steuert die Steuereinrichtung den Aktuator 38 in Abhängigkeit von der durch die Auswerteeinheit 32 ermittelten Federkonstante des Skelettmuskels 20 an. Bei einer solchen Ansteuerung des Aktuators 38 kann das Exoskelett 36 die Funktion des Skelettmuskels 20 unterstützen, wodurch das Gelenk- und Muskelsystem des Benutzers geschont wird.

## Patentansprüche

1. Anziehbares Messgerät (12), mit
einer Messeinrichtung (14), die wenigstens zwei Messeinheiten (16, 18) zum Überwachen eines Skelettmuskels (20) einer Person umfasst, mit einer Halteeinrichtung (26) zum Anbringen der Messeinrichtung (14) an einem den Skelettmuskel (20) aufweisenden Körperteil (10) der Person, und mit einer Auswerteeinheit (32), **dadurch gekennzeichnet, dass** die Messeinheiten (16, 18) jeweils einen Kraftsensor (22, 23) und eine zugeordnete Federeinheit (24, 25) umfassen, wobei sich die Federeinheiten (24, 25) in ihrer Federkonstante voneinander unterscheiden, dass die Messeinheiten (16, 18) derart an dem Körperteil (10) anbringbar sind, dass die Federeinheiten (24, 25) durch einen jeweils anderen Abschnitt des Skelettmuskels (20) spannbar sind, dass
jeder Kraftsensor (22, 23) dazu ausgebildet ist, eine Spannkraft der jeweils zugeordneten Federeinheit (24, 25) zu erfassen, und dass
die Auswerteeinheit (32) dazu ausgebildet ist, in Abhängigkeit von Sensorsignalen der Kraftsensoren (22, 23) eine Federkonstante des Skelettmuskels (20) zu ermitteln.

2. Messgerät (12) nach Anspruch 1, **dadurch gekennzeichnet, dass** wenigstens eine der Federeinheiten (24, 25) eine Metallfeder oder eine Elastomerfeder umfasst.

3. Messgerät (12) nach einem der vorherigen Ansprüche,
**dadurch gekennzeichnet, dass** wenigstens eine der Federeinheiten (24, 25) zylinderförmig, spiralförmig, tellerförmig, balkenförmig, bandförmig oder strangförmig ausgebildet ist.

4. Messgerät (12) nach einem der vorherigen Ansprüche,
**dadurch gekennzeichnet, dass** wenigstens eine der Federeinheiten (24, 25) eine Gasdruckfeder umfasst.

5. Messgerät (12) nach einem der vorherigen Ansprüche,
**dadurch gekennzeichnet, dass** wenigstens einer der Kraftsensoren (22, 23) wenigstens einen kraftabhängigen elektrischen Widerstand, wenigstens eine kraftabhängige elektrische Kapazität, wenigstens eine kraftabhängige elektrische Induktivität oder wenigstens ein Piezoelement umfasst.

6. Messgerät (12) nach einem der vorherigen Ansprüche,
**dadurch gekennzeichnet, dass** die Federeinheiten (24, 25) einen ersten Endabschnitt und einen zweiten Endabschnitt aufweisen, wobei der zugeordnete Kraftsensor (22, 23) durch den ersten Endabschnitt mit der Federeinheit (24, 25) wirkverbunden ist, und wobei die Federeinheit (24, 25) durch den zweiten Endabschnitt mit dem Skelettmuskel (20) wirkverbindbar oder wirkverbunden ist.

7. Messgerät (12) nach einem der Ansprüche 1 bis 5, **dadurch gekennzeichnet, dass** die Federeinheiten (24, 25) einen ersten Endabschnitt und einen zweiten Endabschnitt aufweisen, wobei der zugeordnete Kraftsensor (22, 23) durch den ersten Endabschnitt mit der Federeinheit (24, 25) wirkverbunden ist, und wobei die Federeinheit (24, 25) durch den Kraftsensor (22, 23) mit dem Skelettmuskel (20) wirkverbindbar oder wirkverbunden ist.

8. Messgerät (12) nach einem der vorherigen Ansprüche, **dadurch gekennzeichnet, dass** die Messeinrichtung (14) wenigstens drei Messeinheiten umfasst.

9. Messgerät (12) nach einem der vorherigen Ansprüche, **dadurch gekennzeichnet, dass** die Halteeinrichtung (26) ein Halteband (28), eine Haltemanschette oder eine Haltebandage umfasst.

10. Messgerät (12) nach einem der vorherigen Ansprüche, **dadurch gekennzeichnet, dass** die Halteeinrichtung (26) eine insbesondere starre Tragstruktur (30) umfasst, wobei die Messeinheiten (16, 18) an der Tragstruktur (30) angeordnet sind.

11. Messgerät (12) nach einem der vorherigen Ansprüche, **dadurch gekennzeichnet, dass** sich die Federeinheiten (24, 25) bei bestimmungsgemäßer Anordnung des Messgeräts (12) radial zu dem Körperteil (10) erstrecken.

12. Messgerät (12) nach einem der Ansprüche 1 bis 10, **dadurch gekennzeichnet, dass** sich die Federeinheiten (24, 25) bei bestimmungsgemäßer Anordnung des Messgeräts (12) in Umfangsrichtung des Körperteils (10) entlang des Skelettmuskels (20) erstrecken.

13. Hilfssystem (34), umfassend
ein anziehbares Exoskelett (36) mit wenigstens einem ansteuerbaren Aktuator (38),
wenigstens ein anziehbares Messgerät (12) gemäß einem der Ansprüche 1 bis 12, und
eine Steuereinrichtung, die dazu ausgebildet ist, den Aktuator (38) in Abhängigkeit von durch das Messgerät (12) bereitstellbaren Informationen anzusteuern.

14. Verfahren zum Ermitteln einer Muskelhärte eines Skelettmuskels (20) einer Person, insbesondere mittels eines Messgeräts (12) gemäß einem der Ansprüche 1 bis 12, wobei
wenigstens zwei Federeinheiten (24, 25), die sich in ihrer Federkonstante voneinander unterscheiden, derart an einem den Skelettmuskel (20) aufweisenden Körperteil (10) der Person angebracht werden, dass die Federeinheiten (24, 25) durch einen jeweils anderen Abschnitt des Skelettmuskels (20) spannbar sind, wobei Spannkräfte der Federeinheiten (24, 25) erfasst werden, und wobei
in Abhängigkeit von den erfassten Spannkräften als Muskelhärte eine Federkonstante des Skelettmuskels (20) ermittelt wird.

## Claims

1. Wearable measuring apparatus (12), comprising
a measuring device (14) which has at least two measuring units (16, 18) for monitoring a skeletal muscle (20) of a person, comprising a holding device (26) for attaching the measuring device (14) to a body part (10) of the person, which body part has the skeletal muscle (20), and comprising an evaluation unit (32), **characterized in that** the measuring units (16, 18) each comprise a force sensor (22, 23) and an associated spring unit (24, 25), the spring units (24, 25) differing from each other in their spring constant, **in that** the measuring units (16, 18) can be attached to the body part (10) in such a way that the spring units (24, 25) can be tensioned by a portion of the skeletal muscle which is different in each case (20), **in that**
each force sensor (22, 23) is designed to detect a tension force of the correspondingly associated spring unit (24, 25), and **in that**
the evaluation unit (32) is designed to determine a spring constant of the skeletal muscle (20) depending on sensor signals from the force sensors (22, 23).

2. Measuring apparatus (12) according to claim 1, **characterized in that** at least one of the spring units (24, 25) comprises a metal spring or an elastomer spring.

3. Measuring apparatus (12) according to any of the preceding claims, **characterized in that** at least one of the spring units (24, 25) is cylindrical, helical, planar, bar-shaped, band-shaped or strand-shaped.

4. Measuring apparatus (12) according to any of the preceding claims, **characterized in that** at least one of the spring units (24, 25) comprises a gas spring.

5. Measuring apparatus (12) according to any of the preceding claims, **characterized in that** at least one of the force sensors (22, 23) comprises at least one force-dependent electrical resistor, at least one force-dependent electrical capacitor, at least one force-dependent electrical inductor, or at least one piezoelectric element.

6. Measuring apparatus (12) according to any of the preceding claims, **characterized in that** the spring units (24, 25) have a first end portion and a second end portion, the associated force sensor (22, 23) being operatively connected to the spring unit (24, 25) via the first end portion, and the spring unit (24, 25) being operatively connectable or operatively connected to the skeletal muscle (20) via the second end portion.

7. Measuring apparatus (12) according to any of claims 1 to 5,
**characterized in that** the spring units (24, 25) have a first end portion and a second end portion, the associated force sensor (22, 23) being operatively connected to the spring unit (24, 25) via the first end portion, and the spring unit (24, 25) being operatively connectable or operatively connected to the skeletal muscle (20) via the force sensor (22, 23).

8. Measuring apparatus (12) according to any of the preceding claims, **characterized in that** the measuring device (14) comprises at least three measuring units.

9. Measuring apparatus (12) according to any of the preceding claims, **characterized in that** the holding device (26) comprises a holding strap (28), a holding cuff or a holding bandage.

10. Measuring apparatus (12) according to any of the preceding claims, **characterized in that** the holding device (26) comprises a support structure (30), in particular a rigid support structure, the measuring units (16, 18) being arranged on the support structure (30).

11. Measuring apparatus (12) according to any of the preceding claims, **characterized in that** the spring units (24, 25) extend radially relative to the body part (10) when the measuring apparatus (12) is arranged as intended.

12. Measuring apparatus (12) according to any of claims 1 to 10, **characterized in that** the spring units (24, 25) extend along the skeletal muscle (20) in the circumferential direction of the body part (10) when the measuring apparatus (12) is arranged as intended.

13. Assistance system (34), comprising
a wearable exoskeleton (36) having at least one controllable actuator (38),
at least one wearable measuring apparatus (12) according to any of claims 1 to 12, and
a control device, which is designed to control the actuator (38) depending on information that can be provided by the measuring apparatus (12).

14. Method for determining a muscle hardness of a skeletal muscle (20) of a person, in particular by means of a measuring apparatus (12) according to any of claims 1 to 12, wherein
at least two spring units (24, 25), which differ from each other in their spring constant, are attached to a body part (10) of the person, which body part has the skeletal muscle (20), in such a way that the spring units (24, 25) can be tensioned by a portion of the skeletal muscle (20) which is different in each case, wherein tension forces of the spring units (24, 25) are detected, and wherein,
depending on the detected tensioning forces, a spring constant of the skeletal muscle (20) is determined as muscle hardness.

## Revendications

1. Appareil de mesure (12) pouvant être enfilé, comportant
un dispositif de mesure (14) qui comprend au moins deux unités de mesure (16, 18) pour la surveillance d'un muscle squelettique (20) d'une personne, comportant un dispositif de maintien (26) pour l'application du dispositif de mesure (14) sur une partie du corps (10) de la personne présentant le muscle squelettique (20), et comportant une unité d'évaluation (32), **caractérisé en ce que** les unités de mesure (16, 18) comprennent respectivement un capteur de force (22, 23) et une unité formant ressort (24, 25) associée, dans lequel les unités formant ressorts (24, 25) se différencient l'une de l'autre par leur constante de rappel, **en ce que** les unités de mesure (16, 18) peuvent être appliquées sur la partie du corps (10) de telle sorte que les unités formant ressorts (24, 25) peuvent être tendues par une section respectivement différente du muscle squelettique (20), **en ce que**
chaque capteur de force (22, 23) est configuré pour détecter une force de tension de l'unité formant ressort (24, 25) respectivement associée, **et en ce que**
l'unité d'évaluation (32) est configurée pour déterminer une constante de rappel du muscle squelettique (20) en fonction de signaux de capteurs des capteurs de force (22, 23).

2. Appareil de mesure (12) selon la revendication 1, **caractérisé en ce qu'**au moins l'une des unités formant ressorts (24, 25) comprend un ressort métallique ou un ressort élastomère.

3. Appareil de mesure (12) selon l'une des revendications précédentes, **caractérisé en ce qu'**au moins l'une des unités formant ressorts (24, 25) est réalisée sous une forme de cylindre, de spirale, d'assiette, de poutre, de bande ou de cordon.

4. Appareil de mesure (12) selon l'une des revendications précédentes, **caractérisé en ce qu'**au moins l'une des unités formant ressorts (24, 25) comprend un ressort à gaz.

5. Appareil de mesure (12) selon l'une des revendications précédentes, **caractérisé en ce qu'**au moins l'un des capteurs de force (22, 23) comprend au moins une résistance électrique dépendant de la force, au moins une capacité électrique dépendant de la force, au moins une inductance électrique dépendant de la force ou au moins un élément piézoélectrique.

6. Appareil de mesure (12) selon l'une des revendications précédentes, **caractérisé en ce que** les unités formant ressorts (24, 25) comprennent une première section d'extrémité et une seconde section d'extrémité, dans lequel le capteur de force (22, 23) associé est relié de manière fonctionnelle à l'unité formant ressort (24, 25) par la première section d'extrémité, et dans lequel l'unité formant ressort (24, 25) peut être reliée de manière fonctionnelle ou est reliée de manière fonctionnelle au muscle squelettique (20) par la seconde section d'extrémité.

7. Appareil de mesure (12) selon l'une des revendications 1 à 5,
**caractérisé en ce que** les unités formant ressorts (24, 25) présentent une première section d'extrémité et une seconde section d'extrémité, dans lequel le capteur de force (22, 23) associé est relié de manière fonctionnelle à l'unité formant ressort (24, 25) par la première section d'extrémité, et dans lequel l'unité formant ressort (24, 25) peut être reliée de manière fonctionnelle ou est reliée de manière fonctionnelle au muscle squelettique (20) par le capteur de force (22, 23).

8. Appareil de mesure (12) selon l'une des revendications précédentes, **caractérisé en ce que** le dispositif de mesure (14) comprend au moins trois unités de mesure.

9. Appareil de mesure (12) selon l'une des revendications précédentes, **caractérisé en ce que** le dispositif de maintien (26) comprend une bande de maintien (28), un manchon de maintien ou un bandage de maintien.

10. Appareil de mesure (12) selon l'une des revendications précédentes, **caractérisé en ce que** le dispositif de maintien (26) comprend une structure porteuse (30) en particulier rigide, dans lequel les unités de mesure (16, 18) sont disposées sur la structure porteuse (30).

11. Appareil de mesure (12) selon l'une des revendications précédentes, **caractérisé en ce que** les unités formant ressorts (24, 25) s'étendent radialement par rapport à la partie du corps (10) lorsque l'appareil de mesure (12) est disposé conformément à son utilisation.

12. Appareil de mesure (12) selon l'une des revendications 1 à 10, **caractérisé en ce que** les unités formant ressorts (24, 25) s'étendent le long du muscle squelettique (20) dans la direction périphérique de la partie du corps (10) lorsque l'appareil de mesure (12) est disposé conformément à son utilisation.

13. Système auxiliaire (34), comprenant
un exosquelette (36) pouvant être enfilé et comportant au moins un actionneur (38) pouvant être commandé,
au moins un appareil de mesure (12) pouvant être enfilé selon l'une des revendications 1 à 12, et
un dispositif de commande qui est configuré pour commander l'actionneur (38) en fonction d'informations pouvant être fournies par l'appareil de mesure (12).

14. Procédé permettant de déterminer une dureté musculaire d'un muscle squelettique (20) d'une personne, en particulier au moyen d'un appareil de mesure (12) selon l'une des revendications 1 à 12, dans lequel
au moins deux unités formant ressorts (24, 25), lesquelles se distinguent l'une de l'autre par leur constante de rappel, sont appliquées sur une partie du corps (10) de la personne présentant le muscle squelettique (20) de telle sorte que les unités formant ressorts (24, 25) peuvent être tendues par une section respectivement différente du muscle squelettique (20), dans lequel des forces de tension des unités formant ressorts (24, 25) sont détectées, et dans lequel
une constante élastique du muscle squelettique (20) est déterminée en tant que dureté musculaire en fonction des forces de tension détectées.
